(19) Europäisches Patentamt / European Patent Office / Office européen des brevets

(11) **EP 3 911 218 B1**

(12) **FASCICULE DE BREVET EUROPEEN**

(45) Date de publication et mention de la délivrance du brevet:
**26.02.2025 Bulletin 2025/09**

(21) Numéro de dépôt: **20701312.9**

(22) Date de dépôt: **19.01.2020**

(51) Classification Internationale des Brevets (IPC):
*A61B 5/00* (2006.01)    *G06T 7/00* (2017.01)
*A45D 44/00* (2006.01)

(52) Classification Coopérative des Brevets (CPC):
**A61B 5/0077; A61B 5/445; G06T 7/0012;**
A45D 2044/007; G06T 2207/10024;
G06T 2207/30088

(86) Numéro de dépôt international:
**PCT/EP2020/051207**

(87) Numéro de publication internationale:
**WO 2020/148454 (23.07.2020 Gazette 2020/30)**

(54) **DISPOSITIF POUR LA CARACTÉRISATION ET LA COMPARAISON DE ZONES ÉRYTHÉMALES**

VORRICHTUNG ZUM CHARAKTERISIEREN UND VERGLEICHEN VON ERYTHEMZONEN

DEVICE FOR CHARACTERISING AND COMPARING ERYTHEMAL ZONES

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **18.01.2019 FR 1900477**

(43) Date de publication de la demande:
**24.11.2021 Bulletin 2021/47**

(73) Titulaire: **Pierre Fabre Dermo-Cosmétique**
**81500 Lavaur (FR)**

(72) Inventeurs:
• **JOSSE, Gwendal**
  **31000 Toulouse (FR)**
• **LE DIGABEL, Jimmy**
  **31300 Toulouse (FR)**

(74) Mandataire: **Ipside**
**Tour Trinity**
**1 B Place de la Défense**
**92400 Courbevoie (FR)**

(56) Documents cités:
WO-A2-03/030707     DE-A1- 19 828 497
FR-A1- 3 005 255     US-A1- 2014 088 380
US-A1- 2017 000 406     US-A1- 2017 303 845

• **ANONYMOUS: "Kosmetik - Untersuchsverfahren für Sonnenschutzmittel - In-vivo-Bestimmung des Sonnenschutzfaktors (SSF) (ISO 24444:2010); Deutsche Fassung EN ISO 24444:2010", DIN EN ISO 24444, 1 March 2011 (2011-03-01), pages 1 - 52, XP055515358**

**Description**

L'invention concerne un dispositif pour la caractérisation et la comparaison de zones érythémales de la peau. L'invention est plus particulièrement, mais non exclusivement, adaptée à la détermination d'un indice de protection solaire, ou Facteur de Protection Solaire (FPS), d'un produit photo-protecteur destiné à être appliqué sur la peau.

Technique antérieure

La mise au point et l'évaluation d'un produit cosmétique requièrent de nombreux tests parmi lesquels des tests d'irritation cutanée visant, par exemple, à vérifier l'innocuité d'un produit, ou son indice de protection.

Ces tests sont conduits en exposant la peau, humaine ou animale, au produit ou à une sollicitation telle qu'une exposition au rayonnement ultraviolet, selon différentes modalités, puis à détecter et à caractériser des zones érythémales sur les régions ainsi exposées.

La caractérisation des zones érythémales repose sur une comparaison, soit entre ladite zone érythémale et une peau saine (sans érythème) soit entre deux zones érythémales soumises à des conditions différentes.

La détection d'un érythème et sa caractérisation repose sur l'interprétation des conditions érythémales par un praticien, par exemple un dermatologue.

La détermination du facteur de protection solaire d'un produit photo-protecteur est un exemple de test impliquant la détection et la caractérisation de zones érythémales.

Un produit photo-protecteur est un produit : crème, lait, baume ou poudre contenant tout composant pouvant absorber, réfléchir ou diffuser les rayons UV, qui est destiné à être mis en contact avec la peau humaine. Un tel produit agit par réflexion, absorption ou diffusion d'un rayonnement de manière externe à la peau et n'a aucune action thérapeutique sur celle-ci.

Alternativement, le produit photo-protecteur est un textile.

Le facteur de protection solaire détermine l'accroissement du temps d'exposition aux UV conféré par l'application du produit avant que cette exposition ne produise un érythème, plus communément désigné par « coup de soleil ».

Ainsi, si un sujet s'exposant à un rayonnement UV donné sans protection développe un érythème au bout de 5 minutes d'exposition à ce rayonnement, le même sujet, protégé par l'application d'un produit photo-protecteur ayant un FPS de 10, pourra s'exposer à ce même rayonnement pendant 50 minutes avant de développer un érythème.

La détermination du facteur de protection solaire (FPS) d'un tel produit passe par des tests in vivo comprenant l'exposition de la peau protégée et non protégée d'un sujet à un rayonnement ultraviolet selon des modalités contrôlées.

Ainsi, la détermination du facteur de protection solaire d'un produit appliqué sur la peau est définie par la norme ISO 24444:2010 qui consiste en un test in vivo réalisé sur la peau de plusieurs sujets.

Selon ce test, pour un sujet donné, une première partie de la peau, non protégée, c'est-à-dire sans application du produit photo-protecteur, est exposée, selon des taches sensiblement circulaires, à un rayonnement UV selon des doses croissantes.

Une deuxième partie, adjacente à la première partie, est couverte par le produit protecteur et exposée, selon des taches sensiblement circulaires, à un rayonnement UV également selon des doses croissantes.

Un praticien expérimenté, détermine pour chacune de ces parties la tache pour laquelle la Dose Erythémale Minimale, ou DEM, est atteinte, c'est-à-dire la tache pour laquelle la dose UV reçue par la peau fait apparaître un érythème.

Ainsi, conformément à la norme, la DEM est la plus petite dose de rayonnement ultraviolet (UV) qui produit le premier érythème perceptible non ambigu, présentant des bords réguliers et apparaissant sur la majeure partie du site d'exposition aux UV, dans les 16 h à 24 h suivant l'exposition audit rayonnement UV.

La dose UV correspondant à la DEM de la partie non protégée, dite DEMn, est inférieure à la dose UV correspondant à la DEM de la zone protégée, dite DEMp.

Ainsi, selon cette méthode, le facteur de protection solaire pour un sujet donné, ou FPS individuel (FPSi) correspond au rapport entre la DEM de la zone protégée et la DEM de la zone non protégée pour ce même sujet, et $FPSi=DEMp/DEMn$.

Le FPS du produit est la moyenne arithmétique de toutes les valeurs individuelles valides de FPSi obtenues à partir de tous les sujets de l'essai.

Les conditions relatives à la source d'exposition aux UV, mêlant UVA et UVB, aux zones exposées et à la sélection des sujets sont déterminées par la norme ISO 24444:2010.

Il convient de noter que, selon la norme, la détermination de la zone érythémale utilisée pour la détermination de la DEM est réalisée « en aveugle » par un ou plusieurs praticiens qui n'ont pas participé à l'application du produit et à l'exposition de la zone considérée, par conséquent ne connaissant pas la dose reçue. Cette tâche correspond donc bien à une collecte de données, traitées ultérieurement de manière anonyme, visant à caractériser un produit et non à poser un diagnostic.

Cette méthode d'essai est longue de mise en œuvre et repose sur une série d'interprétations visuelles, nécessairement subjectives, et dépendantes des conditions d'observations conduisant à ces interprétations. La variété des couleurs de peau, les conditions d'éclairage lors de l'analyse et l'acuité visuelle du praticien sont autant de facteurs, parmi d'autres, susceptibles d'influencer les résultats.

La reproductibilité est d'autant plus délicate que la méthode nécessite de déterminer la tache ayant reçu une dose UV, en pratique la plus petite dose, correspondant à l'apparition d'un érythème. Les doses d'exposition étant discrètes et incrémentales, il peut y avoir ambiguïté avec la tache ayant reçu une dose immédiatement inférieure et la tache ayant reçu une dose immédiatement supérieure.

D'autres tests d'irritation cutanée mettant en œuvre différentes zones exposées, notamment les tests innocuité mettant en œuvre une série de patch, reposent sur des interprétations et des comparaisons entre zones exposées et présentent de ce fait également une part interprétative et subjective, susceptible d'introduire de la variabilité dans les résultats.

Le document FR3005255 décrit une méthode photogramétrique dans laquelle une image des zones exposées est normalisée et segmentée, en référence à une mire. La détection de la zone érythémale est réalisée via une méthode par apprentissage. Cette méthode permet de limiter l'influence des conditions environnementales (éclairage, couleur de peau p.ex.) mais reste liée à une interprétation dans le cadre de l'apprentissage.

Le document US2014/0088380 décrit un système d'imagerie multispectral utilisé pour la détection d'ecchymoses et d'ulcères de pression, et utilise à cette fin une acquisition selon un nombre réduit de bandes spectrales.

Le document WO 03/30707 décrit un procédé pour la détermination d'une dose UV érythémale minimale, par une imagerie multispectrale, utilisant une bande spectrale dont l'intensité de la lumière réfléchie est corrélable à la quantité de mélanine dans la peau.

Contrairement au procédé objet de l'invention ces dispositifs et procédés de l'art antérieur ne font pas appel à une opération de démélange spectral permettant d'extraire une signature érythémale dans l'image multispectrale.

Exposé de l'invention

L'objet de l'invention est un dispositif ou système, mettant en œuvre une méthode automatisée ou semi-automatisée, pour la caractérisation et la comparaison de zones érythémales, notamment adapté à la détermination du facteur de protection solaire (FPS) d'un produit photo-protecteur permettant de limiter l'influence de la part interprétative, voire de s'affranchir de toute interprétation.

Ainsi, l'invention concerne un système pour la comparaison de zones érythémales, répondant à la définition de la revendication 5 et pouvant répondre aux caractéristiques des revendications 6 à 8, comprenant une caméra multispectrale pour l'acquisition d'une image multispectrale à des longueurs d'ondes discrètes comprises entre 365 nm et 970 nm des zones comparées, un dispositif de traitement informatique comprenant des moyens de mémoire, d'affichage, de saisie et calcul, apte à récupérer les images acquises par la caméra multispectrale, comprenant un programme de traitement d'image apte à réaliser un démélange spectral des images acquises et à isoler la composante érythémale desdites images, lesdites composantes spectrales correspondant à la modification, imputable à un érythème, du spectre de la peau par rapport au spectre d'une peau saine, et un programme de comparaison des images sur la base de l'intensité des composantes érythémales de chaque image.

Ainsi le système objet de l'invention utilise avantageusement une image multispectrale pour en extraire les composantes propres à une situation érythémale et ainsi faciliter et fiabiliser l'interprétation des images.

L'invention est avantageusement mise en œuvre selon les modes de réalisation et les variantes exposées ci-après lesquels sont à considérer individuellement ou selon toute combinaison techniquement opérante.

Avantageusement, les images acquises par la caméra multispectrale et transmises au système informatique comprennent des métadonnées relatives aux conditions de création des zones érythémales et aux conditions d'acquisition des images. Ainsi l'utilisation de ces métadonnées permet d'automatiser tout ou partie du traitement.

Selon un mode de réalisation particulier, le système objet de l'invention est adapté à la détermination du facteur de protection solaire individuel (FPSi) d'un produit photo-protecteur, les images comparées comprenant les images d'une pluralité de sites correspondant à des expositions à des doses UV croissantes et les métadonnées comprennent tout ou partie des informations relatives au produit testé, aux doses UV correspondant aux différents sites exposés et à la dimension des zones exposées. Ainsi le système objet de l'invention est compatible avec le protocole exposé dans la norme ISO 24444:2010.

Avantageusement, selon ce mode de réalisation particulier, le programme de comparaison comprend la détermination pour chaque image comparée d'une courbe de réponse donnant l'intensité de la composante érythémale en fonction de la dose UV. La détermination de ces courbes de réponse permet de limiter la variabilité des résultats et dans certaines variantes de réalisation de supprimer l'étape de détermination visuelle de la DEM.

Dans une variante du système objet de l'invention adaptée à la détermination du FPSi d'un produit photo-protecteur, les moyens de mémoire du dispositif informatique du système objet de l'invention comprennent un seuil ou une bande d'intensité érythémale fonction de la dose UV, et le programme de comparaison détermine le FPSi par les intersections de cette bande ou de ce seuil avec les courbes de réponse de chaque zone comparée. Cette variante permet la détermination automatique du FPSi.

Dans une autre variante, le programme de comparaison détermine le FPSi par la détermination d'un facteur k d'homothétie minimisant l'écart quadratique entre les courbes de réponse des zones comparées, l'une des courbes étant affectée du facteur k. Ce mode de réalisation permet de déterminer le FPSi sur la base de l'ensemble des sites exposés aux doses UV.

Ainsi, l'invention concerne également un procédé, répondant à la définition de la revendication 1 et pouvant répondre aux caractéristiques des revendications 2 à 4, pour la détermination du FPSi d'un produit de protection solaire à partir de 2 images comprenant chacune une pluralité de taches obtenues par l'exposition d'une peau à des doses UV croissantes, l'une des séries étant obtenue sur une peau sans protection et l'autre sur la peau protégée par le produit, les deux séries faisant apparaître un caractère érythémal sur au moins une des taches, comprenant les étapes consistant à :

a. acquérir une image multispectrale à des longueurs d'ondes discrètes comprises entre 365 nm et 970 nm, de chacune des séries ;

b. procéder pour chacune des images à un démélange spectral pour isoler dans lesdites images les composantes spectrales correspondant à un érythème, lesdites composantes spectrales correspondant à la modification, imputable à un érythème, du spectre de la peau par rapport au spectre d'une peau saine, de sorte à obtenir pour chaque tache de chaque série l'intensité des composantes spectrales correspondant à un érythème ;

c. déterminer à partir du résultat de l'étape b) une courbe de réponse donnant l'intensité des composantes érythémales en fonction de la dose UV pour chacune des séries ;

d. comparer les courbes de réponse ainsi obtenues pour déterminer le FPSi.

Selon un mode de réalisation du procédé objet de l'invention, l'étape d) comprend l'affichage en fausses couleurs des composantes érythémales des images de la zone protégée et de la zone non protégée, et le pointage sur ces images, au moyen des moyens de saisie du dispositif informatique des zones correspondant aux doses érythémales minimales sur chacune des images, reporter l'intensité érythémale correspondant à la dose érythémale minimale ($u_{n1}$) sur la courbe de réponse de la zone protégée pour obtenir la dose $u p_2$ correspondante, reporter l'intensité érythémale correspondant à la dose érythémale minimale ($u_{p1}$) sur la courbe de réponse de la zone non protégée pour obtenir la dose $u_{n2}$, déterminer FPSi par la quantité $(u_{p1} + u_{p2})/(u_{n1} + u_{n2})$. Ce mode de réalisation est en tout point conforme à la norme ISO 24444:2010 mais la détermination de la DEM est plus fiable.

Selon un autre mode de mise en œuvre du procédé objet de l'invention, l'étape d) comprend l'approximation des courbes de réponse par des sigmoïdes $f_n$, $f_p$ et le FPSi est la valeur de $k$ minimisant l'écart quadratique moyen entre les fonctions $f_n$, $f_p$ sur un domaine allant de la plus faible dose UV à la plus importante dose UV d'exposition des zones comparées. Ce mode de réalisation permet une détermination automatique du FPSi en prenant en compte l'ensemble des sites d'exposition.

Avantageusement, l'étape a) comprend l'acquisition d'une zone de peau saine (sans érythème) et l'étape b) de démélange spectral utilise cette acquisition pour la détermination des composantes érythémales.

Le système et le procédé objet de l'invention permettent en outre d'automatiser la caractérisation des zones érythémales, à cette fin ils sont avantageusement utilisés pour effectuer un criblage en vue de la sélection des ingrédients constituant un produit destiné à une application topique.

Description sommaire des dessins

L'invention est exposée ci-après selon ses modes de réalisation préférés, nullement limitatif et en référence aux figures 1 à 7 dans lesquelles :

la figure 1 relative à l'art antérieur représente schématiquement deux zones comprenant une pluralité de sites (taches) exposés à des doses UV croissantes, figure 1A une zone non protégée, figure 1B une zone protégée par un produit photo-protecteur ;

la figure 2 représente schématiquement l'enveloppe des spectres obtenus à partir des images de la caméra multispectrale, figure 2A la comparaison entre une peau sans érythème et une peau irritée, figure 2B le spectre isolé de la réponse érythémale ;

la figure 3 donne un exemple de courbes de réponse en fonction de la dose UV reçue, en séparant la composante peau saine et la composante érythémale ;

la figure 4 montre un exemple de courbes de réponses des composantes érythémales d'une zone protégée et d'une zone non protégée en fonction de la dose UV d'exposition ;

la figure 5 illustre un exemple de détermination du FPSi d'un produit photo-protecteur à partir des courbes de réponse de la figure 4, mettant en œuvre une détection des DEM pour la zone protégée et pour la zone non protégée ;

la figure 6 illustre un exemple de détermination di FPSi d'un produit photo-protecteur à partir des courbes de réponse de la figure 4, mettant en œuvre un seuil ou une bande d'intensité érythémale ;
et la figure 7 représente schématiquement un exemple de réalisation du système objet de l'invention.

**Meilleure manière de réaliser l'invention**

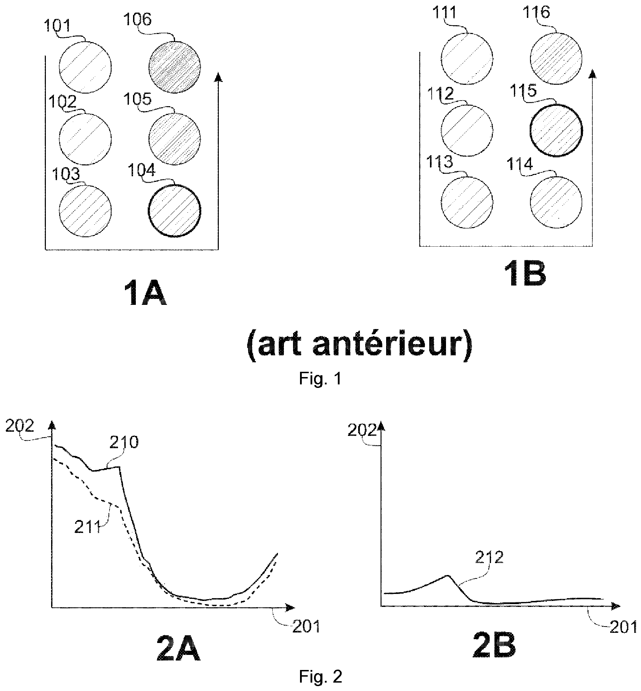

La figure 1, relative à l'art antérieur illustre la méthode de détermination d'une valeur FPSi pour un sujet donné.

Figure 1A, des zones de la peau du sujet (101 à 106), sans protection, dont la localisation est définie par la norme, sont exposées à des doses UV croissantes. Selon un exemple de réalisation, la dose UV est croissante d'un facteur 1,25 d'une zone à l'autre, de sorte que la 6ème zone exposée (106) reçoit une dose 3 fois supérieure à celle de la première zone (101) exposée.

La dose UV reçue par chaque tache est déterminée par le temps d'exposition de la tache à la source UV.

Figure 1B, la même opération est réalisée, sur le même sujet, sur des zones (111 à 116) cette fois protégées par le produit testé.

Pour limiter le nombre d'essais, l'indice de protection du produit est présumé, par exemple sous la forme d'un indice de protection minimum attendu.

Ainsi, si le facteur de protection minimum présumé du produit est par exemple de 12, la première zone (111) de la partie protégée par le produit testé est exposée à une dose 12 fois supérieure à celle à laquelle a été exposée la première zone (101) de la partie non protégée.

Les zones suivantes (112 à 116) sont exposées à des doses croissantes, par exemple selon un facteur 1,25 d'une zone à l'autre.

La procédure décrite ci-avant et qui correspond à la norme ISO 24444:2010 est un préalable à la mise en œuvre du système objet de l'invention qui implémente une méthode d'analyse des résultats, dans le cas de la détermination du FPS d'un produit.

Figure 1A, selon l'art antérieur et en référence à cet exemple, la quatrième zone exposée (104) de la partie non protégée est évaluée comme étant la première, dans l'ordre croissant d'exposition représenté par la flèche brisée de cette figure, à faire apparaître un érythème.

Cette zone correspond ainsi à une dose 4 fois supérieure ($1,25^3$) à la dose reçue pour la première zone (101), et la dose UV correspond à cette quatrième zone est la quantité DEMn.

Figure 1B, toujours selon l'art antérieur et en référence à cet exemple, la cinquième tache (115) de la zone protégée est évaluée comme étant la première dans l'ordre croissant d'exposition à faire apparaître un érythème, et la dose UV reçue par cette cinquième zone (115) de la partie protégée est la quantité DEMp.

Comparativement à la quatrième zone (104) de la partie non protégée et selon cet exemple, cette cinquième zone (115) a reçu une dose UV 15 fois supérieure à celle à laquelle a été exposée cette dernière (1,25x12). Aussi selon cet exemple DEMp/DEMn= FPSi=15.

La même opération est réalisée sur au moins 10 sujets, conformément à la norme, et la valeur FPS du produit est la moyenne arithmétique des FPSi ainsi déterminés.

Le système objet de l'invention met en œuvre une analyse multispectrale des zones exposées et compare selon les modalités des différents modes de mise en œuvre, les résultats issus de cette analyse multispectrale, pour la zone protégée et la zone non protégée.

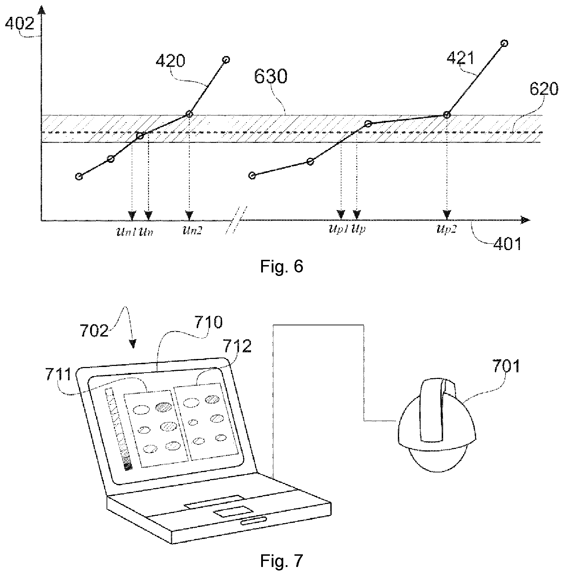

Figure 7, le système objet de l'invention comprend une caméra multispectrale (701), ou tout autre dispositif d'acquisition équivalent, apte à acquérir, en une seule ou en plusieurs prises de vue de la zone d'intérêt, une image en réflectance selon plusieurs longueurs d'ondes isolées.

Ainsi, à la différence d'une image photographique numérique classique qui enregistre les luminances d'une scène selon trois primaires (RVB) les pixels de l'image obtenue par la caméra multispectrale possèdent N primaires correspondant aux N longueurs d'onde d'acquisition de la caméra multispectrale utilisée.

L'imagerie multispectrale permet une définition plus précise de la lumière réfléchie en apportant des nuances de couleur et des informations invisibles à l'œil nu.

Sous le terme générique de « caméra multispectrale » plusieurs technologies d'acquisition sont possibles, mettant en œuvre un éclairage spécifique ou non de la scène.

Avantageusement, la scène analysée, en l'occurrence la peau du sujet sur laquelle l'exposition aux UV a été réalisée, est éclairée par une source lumineuse adaptée au cours de l'acquisition de l'image par la caméra multispectrale, ceci afin d'assurer des conditions calibrées d'éclairage et un éclairement par toutes les longueurs d'onde acquises par la caméra. A titre d'exemple la source lumineuse comprend une sphère d'intégration avec une pluralité de diodes électroluminescentes (LEDS), émettant dans des longueurs d'ondes différentes, correspondant aux longueurs d'onde d'acquisition de la caméra utilisée. Ainsi les conditions d'éclairage sont calibrées et toujours reproductibles.

Selon un exemple de réalisation non limitatif, la caméra réalise une acquisition selon 19 longueurs d'onde discrètes, allant de 365 nanomètres à 970 nanomètres. La plus faible longueur d'onde correspond au domaine ultraviolet et les plus grandes longueurs d'onde (au-delà de 780 nm) au domaine infrarouge. Les longueurs d'onde intermédiaires correspondent au spectre de la lumière visible, du violet au rouge, et la source d'éclairage comprend une pluralité de LEDS dont le spectre d'émission est à l'intérieur du spectre d'acquisition.

A minima, le spectre d'acquisition de la caméra et le spectre d'éclairement doivent permettre l'acquisition selon plusieurs primaires répartis entre 400 nm et 700 nm, zone dans laquelle se situent les pics de réflectance/absorbance de l'hémoglobine, et qui apparaissent (à des intensités différentes) tant sur une peau saine (sans érythème) que sur une peau présentant un érythème.

Les inventeurs ont ainsi pu déterminer que des résultats appropriés et reproductibles comme exposés dans l'exemple en fin de ce document étaient obtenus avec une acquisition multispectrale comprenant au moins 8 longueurs d'onde couvrant 400nm à 700nm.

Ainsi, chaque pixel d'une image enregistrée par la caméra multispectrale du système objet de l'invention est un mélange de plus de 8 primaires, préférentiellement plus de 16 primaires, et selon un exemple de réalisation, comprend jusqu'à 19 primaires.

L'image de chaque tache, acquise directement sur la peau du sujet, et correspondant à une exposition à une dose UV, est traduite en une luminance ou intensité de réflectance, ou alternativement une absorbance, avec absorbance=100-réflectance, et l'analyse de l'image par démélange spectral, telle que décrite ci-après, permet de déterminer la part de cette intensité imputable à une situation érythémale et par suite, de caractériser l'érythème.

La figure 2A compare l'enveloppe du spectre (210) d'une zone de peau présentant un érythème bien développé, donnant l'absorbance (202) en fonction de la longueur d'onde (201), avec l'enveloppe du spectre (211) d'une zone de peau dite saine (sans aucun érythème).

Le spectre de l'image de la zone érythémale est en réalité la combinaison du spectre d'une peau saine, et d'une modification de ce spectre par la présence d'un érythème.

Figure 2B, en retranchant du spectre de la zone érythémale le spectre de la zone de peau saine, il est obtenu le spectre (212) de cette modification imputable à l'érythème. Celui-ci présente notamment une absorbance plus marquée pour les longueurs d'onde voisines de 580 nm, caractéristique de l'absorbance de l'hémoglobine.

Ce spectre (212) constitue en quelque sorte la signature d'un érythème.

Ainsi, l'image d'une zone érythémale est analysée par un traitement de démélange spectral de sorte à en extraire la contribution de l'érythème en termes d'abondances dans les bandes spectrales conformément à cette signature.

Les techniques de démélange spectral sont connues de l'art antérieur et consistent à décomposer le spectre mesuré en un ensemble de composants purs (aussi appelés *endmembers*) et à estimer les proportions (aussi appelées abondances) respectives de chacun de ces composants. En d'autres termes, le spectre observé en chaque pixel est supposé être égal à une combinaison des signatures spectrales d'un certain nombre de composants purs, composants de la peau saine et composants de l'érythème, le but étant d'évaluer la proportion de chacun de ses composants.

Selon un exemple de réalisation la méthode mise en œuvre par le système objet de l'invention utilise une technique de démélange spectral linéaire, en faisant l'hypothèse qu'en chaque pixel le spectre mesuré correspond à la moyenne pondérée (en fonction des abondances) des signatures spectrales des composants purs (peau saine et érythème).

Ainsi, sans entrer dans plus de détails, le démélange spectral constite à exprimer un spectre mesuré $y$ en fonction des composants purs par une équation de type $y=Ma+n$, où $M$ est la matrice des signatures spectrales des composants purs, $a$ le vecteur des abondances et $n$ une perturbation gaussienne, l'inconnue étant le vecteur des abondances a.

Dans le cas de de l'invention, il y a deux composants purs ou enmembers : la peau saine et l'érythème. Un exemple de spectre correspondant à la peau saine (211) est donné figure 2A et un exemple de spectre correspondant à l'érythème (212) est donné figure 2B.

La solution de l'équation est obtenue par un algorithme de régression sous contrainte visant à minimiser les résidus $r$ tels que $r=y-Ma$.

Selon un exemple de mise en œuvre, la méthode mise en œuvre par le système objet de l'invention utilise à cette fin une méthode algorithmique dite parcimonieuse, telle que la méthode SUnSAL, acronyme de « Sparse Unmixing by variable Splitting and Augmented Lagrangian ».

L'application de cette méthode sur une image multispectrale d'une zone de peau exposée ou non à des UV et présentant ou non un érythème permet, en quelque sorte, de classer les pixels de cette image en une classe « peau saine » et une classe « érythème ».

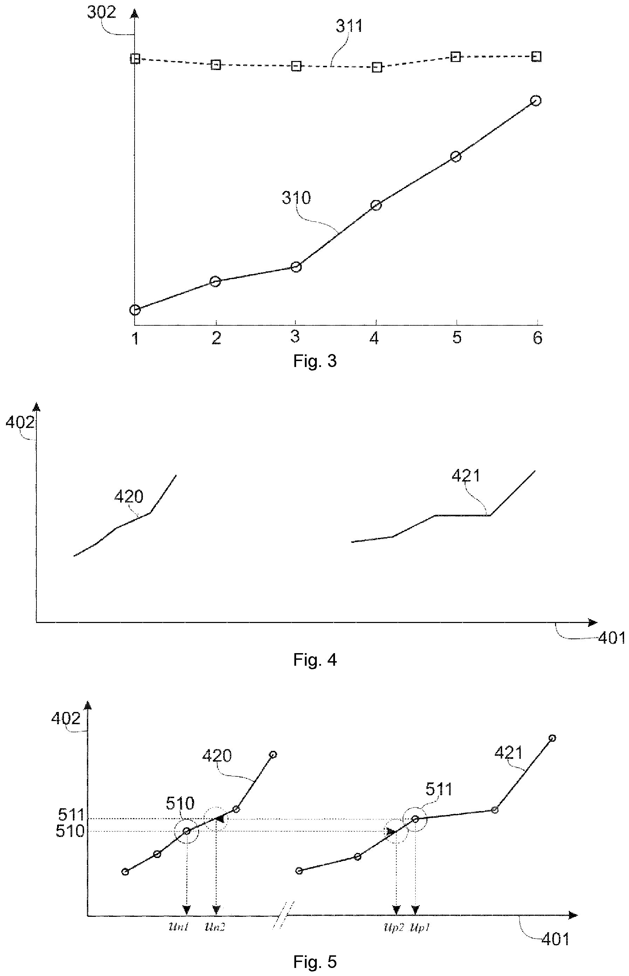

Figure 3, le traitement décrit ci-avant est appliqué sur 6 zones ayant été exposées aux UV selon des doses croissantes de la zone 1 à la zone 6, comme représenté sur la figure 1A ou la figure 1B.

Le traitement d'image pemet de déterminer pour chacune de ces zones l'intensité de l'absorbance (302) correspondant à la peau saine (311) et à l'érythème (310). Cette dernière courbe (310) montre l'abondance relative croissante de la signature spectrale de l'érythème lorsque la dose UV à laquelle la zone a été exposée augmente.

Ce résultat montre la pertinence du démélange spectral appliqué, l'intensité (311) correspondant à la peau saine étant sensiblement constante quelque soit l'intensité de l'érythème.

**EP 3 911 218 B1**

La courbe (310) correspondant à l'intensité de l'absorbance associée à une situation érythèmale est croissante avec le numéro d'ordre de la tache exposée, c'est-à-dire avec la dose UV. Aussi, cette courbe (310) est représentative de la réponse érythémale de la peau en fonction de la dose UV reçue.

Pour la mise en œuvre du système objet de l'invention seule la courbe (310) de réponse érythémale est nécessaire, la courbe (311) correspondant à la peau saine (sans érythème) n'étant représentée sur la figure 3 que pour montrer la pertinence de l'opération de démélange spectral réalisée, ou n'est utilisée que selon des modes de mise en œuvre pefrectionnés.

Figure 4, en appliquant la méthode de démélange spectral sur une acquisition d'image multispectrale réalisée sur la peau sans protection, comme sur la figue 1A, et sur une acquisition réalisée sur une zone protégée comme sur la figure 1B, on obtient deux courbes d'intensité de l'absorbance (402) en fonction de la dose UV (401), la courbe (420) correspondant à la courbe de réponse érythémale pour la zone non protégée, correspondant naturellement à des doses UV inférieures à celles de la courbe (421) de réponse érythémale de la zone protégée, l'érythème apparaissant dans la zone protégée pour des doses UV supérieures.

L'écart entre ces courbes de réponse (420, 421) est représentatif du FPSi du produit testé.

Les deux courbes de réponse (420, 421) correspondant repectivement à la zone sans protection et à la zone protégée, n'ont pas nécessairement exactement la même forme, mais présentent un certain degré d'homothétie.

La courbe $\varphi_n$ (420) est la courbe de réponse de la zone non protégée, et la courbe $\varphi_p$ (421) est la courbe de réponse de la zone protégée.

Ces courbes représent respectivement l'intensité de l'aborbance de la composante érythémale de la peau en fonction de la dose UV à laquelle elle a été exposée. Ainsi la courbe $\varphi_n(u_n)$ donne l'intensité de l'érythème en fonction de la dose UV, $u_n(i)$, reçue par chaque tache $i=1 \dots 6$ de la zone non protégée, et $\varphi_p(u_p)$ donne l'intensité de l'érythème en fonction de la dose UV, $u_p(i)$, reçue par chaque tache $i=1 \dots 6$ de la zone protégée par le produit photoprotecteur.

En assumant que le produit photoprotecteur a un indice de protection minimum attendu $FPS_0$, $u_p(i)=u_n(i).FPS_0$.

Pour déterminer le FPSi, il est considéré que le produit photoprotecteur absorbe, réfléchi ou diffuse une partie du rayonnement UV et ne laisse qu'une proportion $1/k$ de ce rayonnement atteindre la peau.

La réponse de la peau est uniquement contrôlée par la dose UV effectivement reçue. Cette hypothèse est en tout point confomre avec la norme ISO 24444:2010. Ainsi, une zone de peau protégée par le produit photoprotecteur testé et exposée à une dose $u$ reçoit une dose effective de rayonnement $u/k$.

Pour la mise en œuvre du système objet de l'invention en conformiét avec la norme ISO, les doses UV auxquelles sont exposées la partie protégée et la partie non protégée doivent avoir été préalablement sélectionnées de sorte à faire apparaître au moins un érythème correspondant à la définition de la norme ISO 24444:2010 dans chacune de ces zones, c'est-à-dire un érythème perceptible, non ambigu, présentant des bords réguliers et apparaissant sur la majeure partie du site (tache) d'exposition aux UV, dans les 16 heures à 24 heures suivant cette exposition.

Le système objet de l'invention détermine le facteur k, interprétable comme un facteur d'homothétie entre les deux courbes, en comparant les courbes de réponse (420, 421) selon une ou plusieurs méthodes telles qu'exposées ci-après, prises seules ou en combinaison.

Figure 5, plusieurs méthodes sont utilisables à cette fin.

Selon un premier exemple de mise en œuvre, la méthode mise en œuvre par le système objet de l'invention reste en tout point conforme à la norme ISO 24444:2010 et implique la détermination visuelle de la DEMn et de la DEMp. Ainsi, l'intensité (510) des composantes érythémales de la tache correspondant à la DEMn est déterminée sur la zone sans protection, de même que l'intensité (511) correspondant à la DEMp pour la zone protégée. A chacune de ces intensités correspond une dose UV, $u_{n1}$, $u_{p1}$.

En reportant sur la courbe de réponse de la zone non protégée (420) l'intensité (511) correspondant à la DEMp identifiée sur la zone protégée, et en reportant sur la courbe de réponse de la partie protégée (421) l'intensité (510) correspondant à la DEMn, il est possible de définir les valeurs $u_{n2}$ et $u_{p2}$, correspondant respectivement à la dose UV, conduisant sur la zone non protégée à un érythème d'intensité équivalente à celui correspondant à la tache de la zone protégé pour laquelle la DEMp est détectée et vice-versa.

Dans cet exemple de mise en oeuvre FPSi est donné, par exemple, par le ratio $FPSi = (u_{p1}+u_{p2})/(u_{n1}+u_{n2})$.

Comparativement à la méthode de l'art antérieur, cette méthode qui procède en quelque sorte par une double pesée, améliore la précision et la reproductubilité de la détermination de FPSi.

Figure 6, selon un autre exemple de mise en œuvre, un seuil (620) ou une bande (630) d'intensité correspondant à la présence d'un érythème significatif sont déterminés à partir de données statistiques ou par apprentissage. Les intersections du seuil (620) ou de cette bande (630) avec chacune des courbes de réponse permettent, de déterminer une valeur de $k$ et ainsi d'obtenir FPSi.

Selon un mode de réalisation l'algorithme d'apprentissage est préalablement optimisé et paramétré à partir d'une banque d'images de référence annotée avec les scores cliniques des DEM.

Ainsi, en se référant à la figure 6, $FPSI= (u_p/u_n)$ dans le cas d'une estimation par un seuil (620) ou $FPSi = (u_{p1}+u_{p2})/(u_{n1}+u_{n2})$ dans le cas d'une estimation par une bande (630).

La figure 6 représente un seuil (620) et une bande (630) dont les caractéristiques sont constantes en fonction de la dose UV (401).

Selon un mode de réalisation préféré, la métthode mise en œuvre par le système objet de l'invention détermine la valeur de *k* à partir de l'ensemble des courbes de réponse, $\varphi_n(u_n)$ de la partie non protégée (420) et $\varphi_p(u_p)=\varphi_p(k.u_n)$ de la partie protégée (421), soit la valeur de *k* qui minimise l'écart quadratique entre les 2 courbes, ce qui revient à minimiser la quantité :

$$\int_{min(u)}^{max(u)} \left( \boldsymbol{\varphi}_n(u) - \boldsymbol{\varphi}_p(k.u) \right)^2 .du$$

Où *u* est la dose UV.

Pour réaliser cette opération, les courbes de réponse $\varphi_n$, $\varphi_p$ sont approximées par des sigmoïdes $f_n$, $f_p$ et la formiulation mathématique devient :

$$\int_{min(u)}^{max(u)} \left( f_n(u) - f_p(k.u) \right)^2 .du$$

La valeur de *k* minimisant cette quantité est égale à FPSi.

Ainsi, selon ce mode de ralisation, la méthode mise en œuvre par le système objet de l'invention ne nécessite pas la détemination de DEMn et de DEMp, ou un apprentissage quelconque, s'affranchissant ainsi des incertitudes sur cette détermination et réalise l'évaluation du FPSi sur l'ensemble des taches exposées, tant de la partie non protégée que de la partie protégée.

Figure 7, quelque soit le mode de réalisation, le système objet de l'invention comprend une caméra multispectrale (701) et des moyens de traitement (702) par exemple sous la forme d'un micro-ordinateur.

Ledit micro-ordinateur comprend classiquement des moyens de mémoire, des moyens de saisie et de pointage tel qu'un clavier et une souris, des moyens d'affichage tels qu'un ou plusieurs écrans et un micro-processeur.

Le micro-ordinateur (702) est lié informatiquement à la camera multispectrale (701). Selon des variantes de réalisation, cette liaiosn est une liaison filaire, les images acquises par la caméra étant directement transmise au micro-ordinateur, qui selon un exemple de réalisation pilote également l'aquisition réalisée par la caméra, une laison sans fil tel que WIFI® ou Bluetooth® le micro-ordinateur et la caméra mulispectrale étant membres d'un même réseau personnel ou WPAN, ou encore via une connexion réseau de type WAN ou une liaison via le réseau internet. Ce dernier mode de réalisation permet de réaliser l'acquisitition et le traitement des informations dans des sites distants et/ou de manière temporellement décalée de sorte, par exemple, à réaliser des acquisitions et des traitements par lots.

Avantageusement, les images transmises par la caméra multispectrale comprennent un fichier de métadonnées comprenant tout ou partie des informations concernant les conditions d'acquisition de l'image, le produit testé, sous forme codée, le sujet sous forme codée, notamment son angle typologique individuel caractérisant la couleur de sa peau, les doses UV correspondant aux différentes zones exposées, la dimension des zones exposées. Les données relatives au sujet et au produit sont par exemple saisies lors de l'acquisition des images par exemple en scannant un code intelligent, tel qu'un code barre ou un QR code.

Ces informations permettent notamment, via l'analyse de ces données d'associer au sein du micro-ordinateur une acquisition relative à la peau non protégée et une acquisition relative à la peau protégée, puis de regrouper les résultats de traitement dans le but de calculer le FPS.

Les informations relatives au sujet et au produit sont codées, de sorte que l'analyse des résultats est réalisée « en aveugle » conformément à la norme.

Le micro-ordinateur comprend dans ses moyens de mémoire, ou via un accès réseau à une application partagée, un programme de traitement des images issues de la caméra multispectrale pour réaliser le démélange spectrale d'une image, préférentiellement via la méthode SUnSAL, et isoler les composantes imputables à un érythème dans lesdites images.

Un programme de classement permet à partir des résultats du programme de traitement d'image de déterminer la courbe de réponse (420, 421 figures 4 à 6) correspondant à chacune de ces acquisitions.

A la suite de la réalisation de ce traitement le micro-programme en question ou un programme d'affichage complémentaire pemet d'afficher une image (710) en fausses couleurs montrant l'intensité de la composante érythémale sur les différentes taches des zones acquises et de comparer les résultats obtenus sur la zone protégée (711) et sur la zone non-protégée (712).

L'affichage de cette image permet, en autre, de faciliter la détermination de la DEMn et de la DEMp en renforçant visuellement la composante érythémale. Le traitement d'image permet non seulement de restituer une information en intensité de l'érythème, par l'affichage en fausses couleurs, mais également un information spatiale sur l'étendue de la zone érythémale au sein de chaque tache, ce qui facilite la détermination des zones correspondant à la DEMn et à la DEMp.

Ainsi, selon un premier mode de réalisation, l'analyste pointe la zone (tache) correspondant selon lui à la DEMn, puis la zone (tache) correspondant à la DEMp.

Toujours selon ce premier mode de réalisation, après validation, le programme extrait des fichiers de données correspondant les doses UV associées à ces zones et calcule le FPSi en utilisant les courbes de réponse comme présenté plus haut en référence à la figure 5, avant de stocker la valeur ainsi obtenue dans un fichier résulat.

Selon un autre mode de réalisation, le FPSi est déterminé à partir d'un seuil ou d'une bande tel qu'illustré en référence à la figure 6. A cette fin le seuil ou la bande, ou plus avantageusement une pluralité de seuils ou une pluralité de bandes sont stockés dans les moyens de mémoire du dispositif informatique (701) associés par exemples à des caractéristiques de couleur de peau. La sélection du seuil ou de la bande appropriée est réalisée sur la base des métadonnées transmises avec les images.

Selon un autre mode de réalisation, les courbes de réponse de la partie protégée et de la partie non protégée sont approximée par des sigmoïdes et le FPSi du produit est déterminé par un programme visant à minimiser l'écart quadratique moyen entre la courbe de réponse de la zone protégée et la courbe de réponse de la zone non protégée affectée d'un coefficient $k$, $k$ étant le FPSi. Ce mode de réalisation permet d'automatiser la détermination du FPSi par l'analyse des images.

Une partie ou la totalité de ces modes de réalisation sont avantageusement combinés et fournissent ainsi pour une acquisition donnée, une pluaralité de valeurs du FPSi. Selon cet exemple de réalisation combiné, la valeur du FPSi obtenue est considérée valide si la pluralité de valeurs correspondant aux différentes méthodes et comprise dans un interval, par exemple +/- 10 % centré sur la valeur moyenne de la pluralité.

A l'issue du traitement de l'ensemble des résultats valides correspondant au produit testé et stockés dans le fichier résultat sont utilisés pour déterminer le FPS du produit.

La méthode mise en œuvre par le système objet de l'invention est améliorée si en plus des zones exposées à l'irritation, quelque soit l'origine de cette irritation (exposition aux UV ou à un produit en application topique), l'acquisition d'une zone de peau saine, proche de la zone de test, est réalisée. Ainsi, cette acquisition est avantageusement utilisée pour déterminer les composantes spectrales de l'érythème, comme exposé en référence à la figure 2, ou pour contrôler la validité des courbes de réponse comme exposé en référence à la figure 3.

Avantageusement, l'acquisition réalisée sur peau saine est associée à une mire. Ce mode de réalisation permet, entre autre, de déterminer par traitement d'images la couleur du peau du sujet.

Les exemples de réalisation exposés ci-avant sont principalement exposés dans le cadre de la détermination du facteur de protection solaire d'un prodjuit photo-protecteur, cependant tout ou partie des principes et des modes de réaliation exposés sont utilisables pour la caractérisation de zones érythémales dans le cadre d'autres tests d'irritation cutanée, méttant en œuvre des comparaisons de zones érythémales ou des comparaisons entre des zones irritées et des zones saines.

La possibilité de caractérisation automatique des zones soumises au test d'irritation au moyen du dispositif et du procédé objet de l'invention permet pour certaines application de traiter rapidement et de manière fiable un grand nombre de tests.

Ainsi le procédé et le système objets de l'invention sont avantageusement utilisés pour des tests systématiques dans le cadre d'un criblage des ingrédients susceptibles d'entrer dans la composition d'un produit d'application topique, vis-à-vis du pouvoir irritant de ces ingrédients ou de leur effet protecteur.

Exemple

Afin de tester la validité de la méthode une étude de détermination du FPSi d'un produit, référencé P2 dans la norme ISO 24444 : 2010, est réalisée sur 10 sujets.

Chaque sujet est ainsi exposé aux UV sur 2 zones dans le dos, première zone sans agent photo-protecteur, une seconde avec le produit photo-protecteur P2. Ce produit est composé de 3 phases, phase 1 : lanoline 4,5% ; beurre de cacao de Théobroma 2,0% ; monostéarate de glycéryle 3,0% ; acide stéarique 2,0% ; éthylhexyldiméthyle PABA (CAS 21245-02-3) 7,0% ; benzophénone-3 (CAS 131-57-7) 3,0% ; phase 2 : eau 71,6% ; sorbitol (70% de liquide) 5,0% ; triéthanolamine 1,0% ; méthylparabène 0,3% ; propylparabène 0,1% ; phase 3 : alcool benzylique 0,5%. Les données correspondant aux fractions massiques (%).

Chaque zone d'exposition est constituée de 6 spots avec une progression des doses de 1,25 (conformément à la norme). Pour chaque sujet la zone 2 (avec produit photo-protecteur) a été exposée à des doses 14 fois plus grandes que la zone 1. Le système d'exposition utilisé est UV SOLAR LIGHT MULTIPORT BERGER (modèle 601 de 300 W) dont le spectre est conforme à la norme.

24 heures après l'exposition, chaque zone de chaque sujet fait l'objet d'un acquisition multispectrale au moyen du dispositif objet de l'invention comprenant une caméra multispectrale à 19 longueurs d'ondes comprises entre 365 nm et 970 nm, et la valeur des FPSi correspondant à chacun des sujets est déterminé en comparant les courbes de réponse de la partie protégée et de la partie non protégée après démélange spectral et extraction de la composante érythémale.

La répartition des longueurs d'onde d'acquisition de la caméra sont données dans le tableau 1 ci-après

| N° de bande | Nom de la bande | Longueur d'onde [nm] |
|---|---|---|
| 1 | UVA | 365 |
| 2 | Violet | 405 |
| 3 | Indigo | 435 |
| 4 | Bleu | 450 |
| 5 | Bleu | 470 |
| 6 | Cyan | 505 |
| 7 | Vert | 525 |
| 8 | Jaune | 570 |
| 9 | Ambre | 590 |
| 10 | Rouge | 630 |
| 11 | Rouge | 645 |
| 12 | Rouge | 660 |
| 13 | Rouge Foncé | 700 |
| 14 | Proche Infrarouge | 780 |
| 15 | Proche Infrarouge | 850 |
| 16 | Proche Infrarouge | 870 |
| 17 | Proche Infrarouge | 890 |
| 18 | Proche Infrarouge | 950 |
| 19 | Proche Infrarouge | 970 |

Les résultats correspondants du calcul du FPSi sont donnés pour chaque sujet dans le tableau 2 ci-après :

| N° Sujet | FPSi |
|---|---|
| 1 | 11,8 |
| 2 | 17,4 |
| 3 | 7,2 |
| 4 | 18,2 |
| 5 | 23,4 |
| 6 | 16,4 |
| 7 | 15,4 |
| 8 | 17,6 |
| 9 | 15,4 |
| 10 | 17,8 |

La moyenne arithmétique des FPSi fournit la valeur du FPS du produit, en l'occurrence un FPS de 16,06.

Cette valeur de 16,06 correspond à la valeur de référence de la norme pour le produit P2 qui est de 16,1 ; laquelle norme indique en son Annexe C un FPS moyen de 16,1 et compris entre 13,7 et 18,5.

Cet exemple montre la pertinence du procédé objet de l'invention pour la détermination automatique d'un FPSi.

## Revendications

1. Procédé pour la comparaison du facteur de protection solaire individuel, FPSi, d'un produit photo-protecteur à partir de 2 images comprenant chacune une pluralité de taches obtenues par l'exposition d'une peau à des doses UV croissantes, l'une des séries étant obtenue sur une peau sans protection et l'autre sur la peau protégée par le produit, les deux séries faisant apparaître un caractère érythémal sur au moins une des taches, comprenant les étapes consistant à :

a. acquérir une image multispectrale (711, 712) à des longueurs d'ondes discrètes comprises entre 365 nm et 970 nm, de chacune des séries ;

b. procéder pour chacune des images à un démélange spectral pour isoler dans lesdites images les composantes spectrales (212) correspondant à un érythème, lesdites composantes spectrales (212) correspondant à la modification, imputable à un érythème, du spectre de la peau par rapport au spectre d'une peau saine, , de sorte à obtenir pour chaque tache de chaque série l'intensité (402) desdites composantes spectrales (212) correspondant à un érythème ;

c. déterminer à partir du résultat de l'étape b) une courbe de réponse (420, 421) donnant l'intensité desdites composantes spectrales (212) correspondant à un érythème en fonction de la dose UV pour chacune des séries ;

d. comparer les courbes de réponse (420, 421) ainsi obtenues pour déterminer le FPSi.

2. Procédé selon la revendication 1, dans lequel l'étape d) comprend l'approximation des courbes de réponse (420, 421) par des sigmoïdes $f_n$, $f_p$ et où le FPSi est la valeur de k minimisant l'écart quadratique moyen entre les fonction $f_n$, $f_p$ sur un domaine allant de la plus faible dose UV à la plus importante dose UV d'exposition des zones comparées.

3. Procédé selon la revendication 1, dans lequel l'étape a) comprend l'acquisition d'une zone de peau saine (sans érythème) et que l'étape b) de démélange spectral utilise cette acquisition pour la détermination desdites composantes spectrales (212) correspondant à un érythème.

4. Procédé selon la revendication 1, dans lequel l'image multispectrale comprend au moins 8 primaires par pixels correspondant à des longueurs d'onde couvrant 400 nm à 700 nm.

5. Système pour la mise en œuvre du procédé selon la revendication 1, comprenant une caméra multispectrale (701) pour l'acquisition d'une image multispectrale (711, 712) à des longueurs d'ondes discrètes comprises entre 365 nm et 970 nm de zones de peau exposées, un dispositif de traitement informatique (702) comprenant des moyens de mémoire, d'affichage, de saisie et calcul, apte à récupérer les images acquises par la caméra multispectrale, **caractérisé en ce qu'**il comprend un programme de traitement d'image apte à réaliser un démélange spectral des images acquises et à isoler desdites images les composantes spectrales (212) correspondant à un érythème , lesdites composantes spectrales (212) correspondant à la modification, imputable à un érythème, du spectre de la peau par rapport au spectre d'une peau saine, et un programme de comparaison des images sur la base de l'intensité desdites composantes spectrales (212) correspondant à un érythème de chaque image.

6. Système selon la revendication 5, dans lequel les images acquises par la caméra multispectrale (701) et transmises au système informatique (702) comprennent des métadonnées relatives aux conditions de création des zones érythémales et aux conditions d'acquisition des images.

7. Système selon la revendication 5, dans lequel les moyens de mémoire du dispositif informatique comprennent un seuil (620) ou une bande (630) d'intensité érythémale fonction de la dose UV (401), et dans lequel le programme de comparaison détermine le FPSi par les intersections de cette bande (630) ou de ce seuil (620) avec les courbes de réponse (420, 421) de chaque zone comparée.

8. Utilisation du système selon la revendication 5 pour la mise en œuvre du procédé selon la revendication 2, pour effectuer un criblage en vue de la sélection des ingrédients constituant un produit destiné à une application topique.

**Patentansprüche**

1. Verfahren zum Vergleich des individuellen Lichtschutzfaktors, iLSF, eines Lichtschutzprodukts anhand von 2 Bildern, die jeweils eine Vielzahl von Flecken umfassen, die durch die Exposition einer Haut gegenüber steigenden UV-Dosen erhalten werden, wobei eine der Serien auf einer ungeschützten Haut und die andere auf der durch das Produkt geschützten Haut erhalten wird, wobei die beiden Serien auf mindestens einem der Flecken einen erythematösen Charakter zeigen, umfassend die Schritte:

a. Erfassen eines multispektralen Bildes (711, 712) bei diskreten Wellenlängen zwischen 365 nm und 970 nm von jeder der Serien;

b. Durchführen einer spektralen Entmischung für jedes der Bilder, um in den Bildern die einem Erythem entsprechenden spektralen Komponenten (212) zu isolieren, wobei die spektralen Komponenten (212) der einem Erythem zuzuschreibenden Änderung des Spektrums der Haut im Vergleich zum Spektrum einer gesunden Haut entsprechen, sodass für jeden Fleck in jeder Serie die Intensität (402) der einem Erythem entsprechenden spektralen Komponenten (212) erhalten wird;

c. Bestimmen einer Antwortkurve (420, 421) aus dem Ergebnis von Schritt b), die die Intensität der spektralen Komponenten (212), die einem Erythem entsprechen, als Funktion der UV-Dosis für jede der Serien;

d. Vergleichen der so erhaltenen Antwortkurven (420, 421), um den iLSF zu bestimmen.

2. Verfahren nach Anspruch 1, wobei Schritt d) das Approximieren der Antwortkurven (420, 421) durch Sigmoide $f_n$, $f_p$ umfasst und wobei iLSF der Wert von k ist, der die mittlere quadratische Abweichung zwischen den Funktionen $f_n$, $f_p$ über einen Bereich von der niedrigsten UV-Dosis bis zur höchsten UV-Dosis der Exposition der verglichenen Zonen minimiert.

3. Verfahren nach Anspruch 1, wobei Schritt a) das Erfassen einer Zone gesunder Haut (ohne Erythem) umfasst und Schritt b) der spektralen Entmischung diese Erfassung zur Bestimmung der spektralen Komponenten (212) verwendet, die einem Erythem entsprechen.

4. Verfahren nach Anspruch 1, wobei das multispektrale Bild mindestens 8 Primäre pro Pixel umfasst, die Wellenlängen entsprechen, die 400 nm bis 700 nm abdecken.

5. System zur Durchführung des Verfahrens nach Anspruch 1, umfassend eine multispektrale Kamera (701) zur Erfassung eines multispektralen Bildes (711, 712) bei diskreten Wellenlängen zwischen 365 nm und 970 nm von exponierten Hautzonen, ein Gerät zur Computerverarbeitung (702), das Mittel für Speicher, Anzeige, Erfassen und Berechnen umfasst und geeignet ist, die von der multispektralen Kamera erfassten Bilder abzurufen, **dadurch gekennzeichnet, dass** es ein Bildverarbeitungsprogramm umfasst, das geeignet ist, eine spektrale Entmischung der erfassten Bilder durchzuführen und aus den Bildern die einem Erythem entsprechenden Spektralkomponenten (212) zu isolieren, wobei die Spektralkomponenten (212) der einem Erythem zuzuschreibenden Änderung des Spektrums der Haut im Vergleich zum Spektrum gesunder Haut entsprechen, und ein Programm zum Vergleichen der Bilder auf der Grundlage der Intensität der einem Erythem entsprechenden Spektralkomponenten (212) jedes Bildes.

6. System nach Anspruch 5, wobei die von der multispektralen Kamera (701) erfassten und an das Computersystem (702) übertragenen Bilder Metadaten umfassen, die sich auf die Bedingungen für die Entstehung der erythemalen Zonen und die Bedingungen für die Erfassung der Bilder beziehen.

7. System nach Anspruch 5, wobei die Mittel zum Speichern des Geräts für die Datenverarbeitung einen Schwellenwert (620) oder ein Band (630) der Erythemintensität umfassen, die von der UV-Dosis (401) abhängt, und wobei das Vergleichsprogramm den iLSF durch die Schnittpunkte dieses Bandes (630) oder dieses Schwellenwerts (620) mit den Antwortkurven (420, 421) jeder verglichenen Zone bestimmt.

8. Verwendung des Systems nach Anspruch 5 zur Durchführung des Verfahrens nach Anspruch 2, um ein Screening zur Auswahl von Inhaltsstoffen durchzuführen, die ein Produkt bilden, das für eine topische Anwendung bestimmt ist.

**Claims**

1. Method for determining the individual sun protection factor, SPFi, of a photoprotective product from 2 images each of them comprising a plurality of spots obtained by exposing a skin to increasing UV doses, one of the series being obtained on a skin without protection and the other on the skin protected by the product, the two series causing an erythemal character to appear on at least one of the spots, comprising the steps of:

a. acquiring a multispectral image (711, 712) at discrete wavelengths ranging from 365 nanometers to 970 nanometers, of each of the series;

b. carrying out for each of the images a spectral demixing so as to isolate in said images the spectral components (212) corresponding to erythema, said spectral components (212) corresponding to the modification, attributable to erythema, of the spectrum of the skin compared to the spectrum of the healthy skin, so as to obtain for each spot of each of the series the intensity (402) of said spectral components (212) corresponding to erythema;

c. determining from the result of step b) a response curve (420, 421) giving the intensity of said spectral components (212) corresponding to erythema according to the UV dose for each of the series;

d. comparing the response curves (420, 421) thus obtained in order to determine the SPFi.

2. Method according to claim 1, wherein step d) comprises approximating the response curves (420, 421) by sigmoids $f_n$, $f_p$ and where the SPFi is the k value minimizing the mean square deviation between the functions $f_n$, $f_p$ over a range from the lowest UV dose to the highest UV dose of exposure of the compared zones.

3. Method according to claim 1, wherein step a) comprises acquiring a zone of healthy skin (without erythema) and that step b) of spectral demixing uses this acquisition for determining said spectral components (212) corresponding to erythema.

4. Method according to claim 1, wherein the multispectral image comprises at least 8 primaries per pixel corresponding to wavelengths covering 400 nm to 700 nm.

5. System for implementing the method according to claim 1, comprising a multispectral camera (701) for acquiring a multispectral image (711, 712) at discrete wavelengths ranging from 365 nanometers to 970 nanometers of exposed zones of a skin, a computer processing device (702) comprising memory, display, input and calculation means, capable of retrieving the images acquired by the multispectral camera, **characterized in that** it comprises an image processing program capable of performing spectral demixing of the acquired images and isolating from said images the spectral components (212) corresponding to erythema, said spectral components (212) corresponding to the modification, attributable to erythema, of the spectrum of the skin compared to the spectrum of the healthy skin, and a program for comparing the images based on the intensity of said spectral components (212) corresponding to erythema of each image.

6. System according to claim 5, wherein the images acquired by the multispectral camera (701) and transmitted to the computer system (702) comprise metadata relating to the conditions for creating erythemal zones and to the conditions for acquiring the images.

7. System according to claim 5, wherein the memory means of the computer device comprise a threshold (620) or a band (630) of erythemal intensity depending on the UV dose (401), and wherein the comparison program determines the SPFi by the intersections of this band (630) or of this threshold (620) with the response curves (420, 421) of each compared zone.

8. Use of the system according to claim 5 for implementing the method according to claim **2,** for carrying out screening in view of selecting the ingredients constituting a product intended for a topical application.

101 106

102 105

103 104

**1A**

111 116

112 115

113 114

**1B**

(art antérieur)

Fig. 1

202 210

211

201

**2A**

202

212

201

**2B**

Fig. 2

302 ⌐ 311

310

Fig. 3

402 ⌐

420

421

401

Fig. 4

402 ⌐

420

421

511

510

511
510

Un1 Un2    Up2 Up1

401

Fig. 5

Fig. 6

Fig. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 3005255
- US 20140088380 A

- WO 0330707 A

**Littérature non-brevet citée dans la description**

- *CHEMICAL ABSTRACTS*, 21245-02-3

- *CHEMICAL ABSTRACTS*, 131-57-7